Europäisches Patentamt

European Patent Office

Office européen des brevets

(19)

(11) Veröffentlichungsnummer: **0 201 053 B1**

# EUROPÄISCHE PATENTSCHRIFT

(45) Veröffentlichungstag der Patentschrift: **17.04.91**

(51) Int. Cl.⁵: **A61K 39/36**

(21) Anmeldenummer: **86106017.6**

(22) Anmeldetag: **02.05.86**

(54) **Verwendung von Pollenextrakten zur Herstellung von pharmazeutischen Präparaten zur prophylaktischen Behandlung von Allergien.**

(30) Priorität: **06.05.85 CH 1918/85**

(43) Veröffentlichungstag der Anmeldung:
**17.12.86 Patentblatt 86/46**

(45) Bekanntmachung des Hinweises auf die
Patenterteilung:
**17.04.91 Patentblatt 91/16**

(84) Benannte Vertragsstaaten:
**AT BE DE FR GB IT LU NL SE**

(56) Entgegenhaltungen:
**DE-A- 1 467 750**

(73) Patentinhaber: **Cernitin S.A.**

**CH-6911 Barbengo(CH)**

(72) Erfinder: **Lewenstein, Ari**
**Via Marco da Carona 5**
**CH-6900 Lugano(CH)**

(74) Vertreter: **Blum, Rudolf Emil Ernst et al**
**c/o E. Blum & Co Patentanwälte Vorderberg 11**
**CH-8044 Zürich(CH)**

## Beschreibung

### HINTERGRUND DER ERFINDUNG:

Prophylaktische Verfahren zur Behandlung von Allergien, beispielsweise Heuschnupfen oder allergischem Asthma, sind bereits bekannt. Diese Behandlungen sind jedoch langwierig, zeitraubend und kostspielig, weil Injektionsbehandlungen mit steigenden Dosierungen des Allergens, beispielsweise Pflanzenpollen, nötig sind oder weil mehrere Injektionen spezieller Fraktionen des menschlichen Gammaglobulines nötig sind.

Ziel der vorliegenden Erfindung war es, ein neues pharmazeutisches Präparat zur Verfügung zu stellen, das zur prophylaktischen Behandlung von Allergien eingesetzt werden kann und das oral verabreicht oder eventuell topisch angewendet werden kann.

### BESCHREIBUNG DES STANDES DER TECHNIK:

Prophylaktische therapeutische Verfahren zur Behandlung von Allergien, beispielsweise Heuschnupfen oder allergischem Asthma, sind bereits bekannt. Bei den bisher bekannten prophylaktischen Behandlungsverfahren werden im allgemeinen Hauttests mit üblichen Allergenen, wie zum Beispiel Pflanzenpollen, Tierhaaren, Vogelfedern und ähnlichem, durchgeführt, um festzustellen, auf welche Allergene der Organismus des Patienten reagiert Anschliessend erfolgt dann eine desensibilisierende Injektionsbehandlung mit immer stärker ansteigenden Dosierungen des Allergen, beispielsweise der Pollen. Diese desensibilisierende Behandlung ist nicht nur äusserst kostenintensiv und langwierig für den Patienten, sondern sie kann auch zu unerwünschten Nebenerscheinungen führen, insbesondere deshalb, weil die zur Desensibilisierung verwendeten Präparate nicht ausreichend rein sind. Falls der Patient während der prophylaktischen Behandlung unerwartet mit dem Allergen in Berührung kommt, kann eine sehr starke Reizreaktion, und im Extremfall, ein anaphylaktischer Schock auftreten.

Aufgrund dieser Nachteile wird in jüngster Zeit zur prophylaktischen Behandlung eine allgemeine Desensibilisierung des Patienten mit speziellen Fraktionen an menschlichem Gammablobulin vorgenommen. In diesem Falle wird nicht versucht, zunächst mit Hauttests zu prüfen, auf welche Substanzen der Patient wirklich allergisch ist, sondern es erfolgt etwa drei Monate vor dem erwarteten Ausbruch der Allergie die Injektionsbehandlung mit der Gammaglobulinfraktion. Diese Behandlungsweise ist weit weniger gefährlich als die vorher beschriebene Desensibilisierung, sie ist jedoch ebenfalls kostenaufwendig, weil eine grosse Anzahl an Injektionsbehandlungen nötig ist. Ferner ist es auch nicht möglich, die erforderliche Dosierung zu erkennen und, falls eine zu geringe Dosierung gewählt wurde, tritt zum gegebenen Zeitpunkt die Allergie in üblicher Stärke auf.

Es ist ferner bekannt, dass Pollen eine Vielzahl an Substanzen enthält, die als Nährstoffe wertvoll sind und deshalb sind im Handel viele diätetische Produkte und kosmetische Produkte, die Pflanzenpollen enthalten, erhältlich. Aufgrund der hochmolekularen Proteine, die in der Pollenhülle anwesend sind, können jedoch Produkte, die Pflanzenpollen enthalten allergische Reaktionen bei den sie verwendenden Personen hervorrufen, wenn die fragliche Person gegenüber dem Allergen empfindlich ist.

In der USA Patentschrift Nr. 3,360,437 von C.G. Carlsson et al. wird ein Verfahren zur Gewinnung von wichtigen Bestandteilen der Pollen beschrieben, wobei die erhaltenen Produkte keine Proteinbestandteile hohen Molekulargewichts der Pollen enthalten, die Allergien hervorrufen können. Nach diesem Verfahren werden die wichtigen Bestandteile der Pollen erhalten, indem man eine Extraktion durchführt, welche die folgenden Arbeitsschritte umfasst:

(a) eine Extraktion der Pollen mit einer wässrigen Lösung von Aethanol oder Aceton,

(b) ein Abbau des Pollen enthaltenden Extraktes mit dem Mikroorganismus Mucor hiemalis während etwa 48 Stunden und

(c) eine Abtrennung der Lösung von den Pollenrückständen.

Gemäss einem weiteren Verfahren, das in dieser USA Patentschrift beschrieben wird, werden wichtige Bestandteile der Pollen gewonnen, indem man ein Extraktionsverfahren durchführt, welches die folgenden Arbeitsschritte umfasst:

(a) eine Extraktion der Pollen mit einer wässrigen Lösung von Aethanol oder Aceton,

(b) eine Abbaubehandlung der in dem Extraktionsmedium befindlichen Pollen mit dem Mikroorganismus Mucor hiemalis während etwa 48 Stunden,

(c) eine Abtrennung der danach erhaltenen Lösung von den Pollenrückständen,

(d) eine Extraktion der Pollenrückstände mit einem flüchtigen lipid-löslichen Lösungsmittel, das Aethyläther oder Aceton ist und

(e) eine Abtrennung des erhaltenen Extraktes aus dem Pollenrückstand.

Auch eine kombinierte Extraktion der Pollen mit einer wässrigen Lösung von Aethanol und Aceton und eine weitere Extraktion mit einem flüchtigen lipidlöslichen Lösungsmittel aus der Gruppe Aethyläther und Aceton, wird in diesem U.S.A. Patent beschrieben.

Der Abbau der hochmolekularen Proteine des Pollens mit dem Schimmelpilz der Gattung Mucor führt bei diesem dort beschriebenen Verfahren dazu, dass die erhaltenen Produkte frei von Substanzen sind, die Allergien hervorrufen können, wenn Personen, die gegenüber den entsprechenden hochmolekularen Proteinen empfindlich sind, die fraglichen Produkte konsumieren (Es sei in diesem Zusammenhang auf Spalte 1, Zeilen 60 - 65, und Spalte 2, Zeilen 58 - 64, des fraglichen USA Patentes verwiesen).

Verfahren, welche dem im USA Patent beschriebenen Arbeitsverfahren ähnlich sind, und bei denen verschiedene weitere Stämme von Mucor zum Abbau der Pollen verwendet werden, sind in der österreichischen Patentschrift Nr. 255 643 und der deutschen Patentschrift Nr. 1 467 750 beschrieben.

Pollenextrakte, die nach diesen Verfahren des Standes der Technik unter Verwendung des wässrigen Extraktionsmediums oder des nicht wässrigen Extraktionsmediums hergestellt wurden oder Mischungen aus den Extrakten, die unter Verwendung des wässrigen Mediums und des nicht wässrigen Mediums erzeugt wurden, sind im Handel erhältlich. Derartige Produkte werden als Stärkungsmittel, zur Behandlung von Prostataleiden, zur Beschleunigung der Heilung von Wunden und Knochenbrüchen und als entzündungshemmende Mittel eingesetzt. Die fraglichen Produkte werden oral oder topisch verabreicht, beispielsweise als Salben oder ähnliche Produkte zur Wundheilung.

Wie bereits erwähnt wurde, wurden die fraglichen Produkte, bevor sie auf den Markt gebracht wurden, genau getestet, um nachzuweisen, dass die Produkte, die den Pollenextrakt enthalten, der im westenlichen frei von hochmolekularen Proteinen ist, keine Allergien hervorrufen.

Es wurde nun völlig unerwartet festgestellt, dass diese im Handel erhältlichen Produkte und ähnliche Produkte, die als aktiven Bestandteil einen Pollenextrakt enthalten, zur prophylaktischen Behandlung von Allergien herangezogen werden können.

Die vorliegende Erfindung betrifft dementsprechend die Verwendung von Pollenextrakten, die nicht mehr als höchstens 5 Gew.-% an hochmolekularen Proteinen enthalten, zur Herstellung von pharmazeutischen Präparaten zur prophylaktischen Behandlung von Allergien.

## BESCHREIBUNG DER ERFINDUNG

Der Gegenstand der vorliegenden Erfindung ist die Verwendung von Pollenextrakten, die nicht mehr als höchstens 5 Gew.-% an hochmolekularen Proteinen enthalten und die durch folgendes Verfahren erhältlich sind:

Extraktion der Pollen mit einem wässrigen Extraktionsmittel,

Entfernung der hochmolekularen Proteine, vorzugsweise durch Abbau derselben, bis zur Erreichung des oben angegebenen Maximalgehaltes an diesen, sowie

Extraktion der Pollen mit einem nicht wässrigen Extraktionsmittel,

zur Herstellung von pharmazeutischen Präparaten zur prophylaktischen Behandlung von Allergien.

Speziell vorteilhaft ist es, zur Herstellung der pharmazeutischen Präparate zur prophylaktischen Behandlung von Allergien, solche Pollenextrakte zu verwenden, die frei von hochmolekularen Proteinen sind.

Die durch die erfindungsgemässe Verwendung von Pollenextrakten hergestellten pharmazeutischen Präparate sind vorzugsweise Präparate zur oralen Verabreichung. Die fraglichen pharmazeutischen Präparate können jedoch auch so formuliert werden, dass sie zur topischen Anwendung geeignet sind, beispielsweise in Form von Lösungen oder Salben, die zur Verwendung in der Nase oder in den Augen vorgesehen sind, als versprühbare Präparate, die aus einer Sprühdose mit einem Sprühmittel freigesetzt werden, wobei derartige Sprays verwendet werden um Heuschnupfen oder allergisches Asthma prophylaktisch zu behandeln, oder sie können als Salben, Lotionen oder ähnliche Produkte formuliert sein, die auf die Haut aufgetragen werden. Gegebenenfalls können jedoch die pharmazeutischen Präparate auch so formuliert werden, dass sie durch Injektion verabreichbar sind.

Bei der erfindungsgemässen Verwenduns wird als Pollenextrakt vorzugsweise der Extrakt von Pollen eingesetzt, die von der Pflanzenfamilie der Nadelgehölze insbesondere der Pinaceae, der Getreidearten und Gräser, nämlich der Gramineae, insbesondere der Klasse der Framineae, stammen, oder es wird ein Extrakt einer Mischung von Pollen dieser beiden Pflanzenfamilien oder einer Mischung von Pollen mindestens einer dieser Pflanzenfamilien mit Pollen aus anderen Pflanzenfamilien, insbesondere der Familien der Liliengewächse, der Korbblütler, der Rosengewächse, der Weidengewächse, Birkengewächse und Ulmengewächse verwendet.

Als Beispeile für Pollen aus der Klasse der Getreidearten oder Gräser, deren Extrakt zur Durchführung des erfindungsgemässen Verfahrens gut geeignet ist, seien diejenigen von Mais mit der lateinischen Bezeichnung Zea mays, von Roggen mit der lateinischen Bezeichnung Secale cereale und von Wiesenlieschgras, mit der lateinischen Bezeichnung Phleum pratense, genannt.

Zur Herstellung der pharmazeutischen Präparate kann man den mit einem wässrigen Extraktionsmittel gewonnenen Extrakt oder den mit einem nicht wässrigen Extraktionsmittel gewonnenen Extrakt verwenden oder man kann auch eine Mischung aus dem mit einem wässrigen Extraktionsmittel und dem mit einem nicht wässrigen Extraktionsmittel gewonnenen Extrakt einsetzen.

Der in den pharmazeutischen Präparaten verwendete Pollenextrakt wird vorzugsweise hergestellt, indem man frisch geerntete oder getrocknete Pflanzenpollen mit einem wässrigen Extraktionsmittel extrahiert, anschliessend die vorhandenen hochmolekularen Proteine vollständig oder bis zu einem Maximalgehalt von 5 Gew.-%, bezogen auf das Trockengewicht, durch Einwirkung von Mikroorganismen zu niedrigmolekularen Proteinen oder Aminosäuren abbaut, das wässrige Medium abtrennt und den Pollenrückstand mit einem nicht wässrigen Medium weiter extrahiert.

Bei diesem Verfahren kann man den durch die Extraktion mit dem wässrigen Medium nach der Behandlung mit dem Mikroorganismus gewonnenen wässrigen Extrakt oder den nach der Extraktion mit dem nicht wässrigen Extraktionsmittel gewonnenen Extrakt einengen indem man das Lösungsmittel abdampft, wobei man dabei einen entsprechenden höher konzentrierten Extrakt oder einen Trockenrückstand erhält, und diese Produkte werden dann als Wirkstoff zur Herstellung der pharmazeutischen Präparate eingesetzt.

Speziell bevorzugt ist es, ein Gemisch aus dem mit dem wässrigen Extraktionsmittel gewonnen und dem mit dem nichtwässrigen Extraktionsmittel gewonnenen Extrakt als Wirkstoff in den pharmazeutischen Präparaten einzusetzen und zwar speziell bevorzugt ein Gemisch mit einem gewichtsmässig höheren Anteil an dem Trockenrückstand des mit dem wässrigen Extraktionsmittel gewonnenen Extraktes im Vergleich zu dem mit dem nichtwässrigen Extraktionsmittel gewonnenen Extrakt.

Als besonders günstig erwies es sich dabei, eine Mischung einzusetzen, die berechnet als Trockengewicht des Extraktes aus 10 bis 30 Gewichtsteilen, beispielsweise 20 Gewichtsteilen, des mit dem wässrigen Extraktionsmittel gewonnenen Extraktes und einem Gewichtsteil, bezogen auf das Trockengewicht des Extraktes des mit dem nichtwässrigen Extraktionsmittel gewonnenen Extraktes, besteht.

Zur Herstellung der Wirkstoffe der pharmazeutischen Präparate werden zweckmässigerweise die frisch geernteten oder vorgetrockneten Pflanzenpollen zunächst mit dem wässrigen Extraktionsmittel während 36 Stunden bis 60 Stunden bei einer Temperatur im Bereich von 28° C bis 35° C, vorzugsweise 30 - 32° C, extrahiert. Im allgemeinen werden pro kg an Pflanzenpollen etwa 4 bis 6 kg des wässrigen Extraktionsmittels eingesetzt.

Als wässriges Extraktionsmittel wird vorzugsweise eine Mischung aus Wasser mit einem gewichtsmässig geringeren Anteil an einem vollständig mit Wasser mischbaren organischen Lösungsmittel eingesetzt, beispielsweise an einen niederen Alkohol oder niederen Keton, wie zum Beispiel Aethylalkohol oder Aceton. Das wässrige Extraktionsmittel weist im allgemeinen einen Gehalt von 2 bis 30% des mit Wasser mischbaren organischen Lösungsmittel auf, wobei der Rest auf 100 Gew.-% aus Wasser, vorzugsweise destilliertem Wasser, besteht.

Als Beispiele für gut geeignete wässrige Extraktionsmittel sei eine 20 Gew.-%-ige wässrige Lösung von Aethylalkohol oder eine 5 Gew.-%-ige wässrige Lösung von Aceton genannt.

Nach dieser Vorextraktionsbehandlung setzt man Mikroorganismen zu und belässt die Pollen in dem wässrigen Extraktionsmedium unter Rühren während weiterer 36 bis 60 Stunden bei einer Temperatur von 15 - 35° C. Während dieser Zeit erfolgt die Fermentierung der äusseren Pollenhüllen. Die auf den Pollen anwesenden Mikroorganismen bauen die hochmolekularen Proteine zu niedrigmolekularen Proteinen oder freien Aminosäuren ab.

Als Allergen wirkende Bestandteile, die in den als Ausgangsmaterial eingesetzten Pollen enthalten sein können, sind hochmolekulare Proteine. Diese werden durch die genannte Einwirkung der Mikroorganismen zumindestens so weit abgebaut, dass das nach der Fermentation erhaltene Produkt keine allergene Wirkung zeigt.

Als Mikroorganismen werden für die Durchführung der Fermentation mit Vorteil Pilze verwendet. Pilze, die für diese Fermentation gut geeignet sind, sind solche der Gattung Mucor. Als Beispiel für derartige Pilze, welche den Abbau hochmolekularer Proteine bewirken, seien genannt: Mucor glomerata; Mucor luteus; Mucor mucedo; Mucor flavus, Mucor corticolus, Mucor brunneus, Mucor hiemalis oder Mucor alpinus.

Nach dieser Fermentationsbehandlung erfolgt eine Abtrennung der Pollen von dem wässrigen Extraktionsmedium, beispielsweise durch Filtrieren und das erhaltene Filtrat stellt eine klare Lösung dar und durch Analyse konnte gezeigt werden, dass es unter anderem Vitamine, verschiedene Zuckerarten, Desoxyriboside, niedrigmolekulare Proteine, Aminosäuren und Spurenelemente enthält.

Der nach der Abtrennung des wässrigen Extraktionsmediums zurückbleibende Pollenrückstand wird dann mit einem nichtwässrigen organischen Extraktionsmittel vermischt und weiter extrahiert, im allgemeinen ebenfalls während etwa 36 - 60 Stunden, und diese Extraktionsbehandlung erfolgt im allgemeinen bei Zimmertemperatur.

Zweckmässigerweise werden pro kg der als Ausgangsmaterial eingesetzten Pollen 3 - 6 kg. des organischen nichtwässrigen Extraktionsmittels eingesetzt.

Als organisches nichtwässriges Extraktionsmittel werden vorzugsweise niedrige Ketone, Dialkyläther, Carbonsäureester oder Kohlenwasserstoffe, vorzugsweise aliphatische oder cycloaliphatische Kohlenwasserstoffe, verwendet. Als Beispiele für verwendbare Extraktionsmittel seien Aceton, Diäthyläther, niedrig siedende Erdölfraktionen, sowie Hexan oder Heptan genannt.

Nach der Behandlung mit dem organischen Extraktionsmittel wird dieses von dem Pollenrückstand abgetrennt, beispielsweise durch Filtrieren oder durch Abschleudern. Der gewonnene Extrakt ist eine klare Lösung und er enthält die lipoidlöslichen Stoffe der Pflanzenpollen, wie zum Beispiel Sterole, Wachse, Chlorophylle und ähnliches.

Vorzugsweise werden aus dem mit dem wässrigen Extraktionsmittel gewonnenen Extrakt und auch aus dem mit dem nichtwässrigen Extraktionsmittel gewonnen Extrakt die Lösungsmittel abgedampft, gegebenenfalls unter Vakuum, wobei man entsprechende Konzentrate, bzw. Trockenrückstände erhält. Diese können direkt als Wirkstof zur Herstellung des pharmazeutischen Präparates eingesetzt werden, sie stellen also das Material dar, das für die erfindungsgemässe Verwendung vorgesehen ist.

Bei der Erzeugung der pharmazeutischen Präparate ist es möglich, durch eine spezielle Auswahl der Mischung an Pollen, die als Ausgangsmaterial zur Herstellung dieser Präparate herangezogen werden, solche pharmazeutischen Präparate herzustellen, die zur prophylaktischen Behandlung spezieller Allergien besonders vorteilhaft geeignet sind.

Patienten, die auf Pflanzenpollen allergisch reagieren, reagieren im allgemeinen auf die Pollen von Windblütlern allergisch, weil bekanntlich solche Pflanzen, die durch Windbestäubung befruchtet werden, besonders hohe Mengen an Pollen freisetzen müssen, um die Befruchtung zu gewährleisten. Ferner ist bekannt, dass von den Windblütlern wiederum diejenigen mit kleinen Blütenpollen besonders häufig allergische Reaktionen hervorrufen. Als Beispiele für bekannte, als Allergen wirkende Pollen seien diejenigen von Haselnüssen, Birken, Erlen, Eschen, Edelkastanien, Gräsern und auch Wegerich genannt. Obwohl eine Anzahl von Patienten auf mehrere unterschiedliche Pollenarten allergisch reagiert so gibt es doch viele Patienten, die nur auf eine bestimmte Pollenart allergisch reagieren. Durch die entsprechende Auswahl der Pollen in dem zur Herstellung der erfindungsgemässen pharmazeutischen Präparate herangezogenen Ausgangsmaterial, ist es möglich, solche prophylaktisch wirksame pharmazeutische Präparate zu erhalten, die gegen bestimmte spezifische Allergien besonders gut wirksam sind.

Durch die erfindungsgemässe Verwendung werden bevorzugt solche Präparate hergestellt, die als Wirkstoff eine Mischung aus einem gewichtsmässig grösseren Anteil, bezogen auf das Trokkengewicht des Extraktes, des mit dem wässrigen Extraktionsmittel gewonnenen Pollenextraktes und ferner einen gewichtsmässig kleineren Anteil, bezogen auf das Trockengewicht des Extraktes, des mit dem nichtwässrigen Extraktionsmittel gewonnenen Pollenextraktes enthalten. Speziell bevorzugt sind von diesen pharmazeutischen Präparatensolche, die zur oralen Verabreichung geeignet sind, wobei diese Präparate zweckmässigerweise so formuliert werden, dass sie vom Verbraucher leicht in einzelne Dosierungseinheiten unterteilt werden können.

Speziell bevorzugte derartige zur oralen Verabreichung geeignete, durch die erfindungsgemässe Verwendung hergestellte pharmazeutische Präparate enthalten pro Dosierungseinheit, beispielsweise pro Tablette oder Kapsel, 50 - 70 mg, bezogen auf das Trockengewicht des Extraktes, beispielsweise 60 mg, bezogen auf das Trockengewicht des Extraktes, an dem mit dem wässrigen Extraktionsmittel gewonnenen Pollenextrakt und ferner 2 - 4 mg, bezogen auf das Trockengewicht des Extraktes, beispielsweise 3 mg, bezogen auf das Trockengewicht des Extraktes, an dem mit dem nicht wässrigen Extraktionsmittel gewonnenen Extrakt.

Anhand der nachfolgenden Beispiele wird die erfindungsgemässe Verwendung zur Herstellung der pharmazeutischen Präparate zur prophylaktischen Behandlung von Allergien, bzw. die Testergebnisse, die bei der Testung derartiger Präparate erhalten wurden, näher erläutert. Die Beispiele sollen jedoch in keiner Weise als den Erfindungsgedanken einschränkend, betrachtet werden.

### Beispiel 1

120 kg Pollen von Roggen mit der lateinischen Bezeichnung Secale cereale werden mit 600 kg einer 20 vol.-prozentigen Lösung von Aethylalkohol in Wasser behandelt. Man rührt die Pollen während 48 Stunden bei einer Tem,peratur von 30 - 32° C. Nach dieser Vorbehandlung setzt man Mucor alpinus zu und rührt bei 30° C während 20 - 30 Stunden.

Das wässrige Medium wird von den Pollen abfiltriert. Es stellt eine klare Lösung dar, aus der das Lösungsmittel unter Vakuum abgedempft wird, wobei Trockenrückstand zurückbleibt, der erfindungsgemäss zur Herstellung der pharmazeutischen Präparate zur prophylaktischen Behandlung von Allergien Verwendung findet.

Die nach dem Abfiltrieren des wässrigen Extraktionsmediums zurückbleibenden Pollenrückstände werden mit 400 kg Aceton unter Rühren während 48 Stunden bei Zimmertemperatur einer erneuten Extraktion unterzogen. Nach dem Abschleudern der Pollenrückstände erhält man eine klare Lösung, aus der durch Abdampfen unter Vakuum ein Trockenrückstand gewonnen wird, der ebenfalls erfindungsgemäss als Wirkstoff zur Herstellung der pharmazeutischen Präparate zur prophylaktischen Behandlung von Allergien eingesetzt wird.

Gegebenfalls können die abgeschleuderten Pollenrückstände erneut mit dem wässrigen Lösungsmittel und anschliessend mit dem Aceton behandelt werden, um weitere Ausbeuten an wasserlöslichen Wirkstoffen, bzw. lipoidlöslichen Wirkstoffen, zu erhalten.

Beispiel 2

Es wurden Tabletten formuliert, die pro Tablette 60 mg des Trockenrückstandes enthielten, der gemäss Beispiel 1 unter Verwendung des wässrigen Extraktionsmittels erhalten wurde, und ferner 3 mg des Trockenrückstandes enthielten, der nach dem Verfahren gemäss Beispiel 1 unter Verwendung von Aceton als Extraktionsmedium erhalten wurde.

Diese Tabletten wurden zur prophylaktischen Behandlung von Heuschnupfen und Allergien bei erwachsenen weiblichen und männlichen Patienten und auch bei Kindern herangezogen. Die Verabreichung erfolgte in den Monaten Februar bis Ende April. An die Kinder unter 10 Jahren wurde täglich drei Tabletten verabreicht, und zwar je eine morgens, mittags und abends, und die Erwachsenen erhielten täglich zwölf Tabletten, und zwar je vier morgens, mittags und abends.

Sämtliche getesteten Patienten waren solche, bei denen in früheren Jahren sehr schwere pollenbedingte Allergien diagnostiziert wurden. Bei den erwachsenen Patienten handelte es sich um Frauen und Männer, die entweder unter sehr starken Symptomen des Heuschnupfens gelitten hatten, wie dauernde Niessreize, laufende Nase, tränende Augen, geschwollenes Gesicht und geschwollene Schleimhäuteoder die unter einem durch Pollenallergie hervorgerufenen Asthma litten. Die getesteten Kinder, beispielsweise ein achtjähriger Knabe, hatten ausnahmslos in vorherigen Jahren unter starken durch Pollenallergie hervorgerufenen asthmatischen Zuständen gelitten.

Es zeigte sich, dass sämtliche Patienten, die der entsprechenden prophylaktischen Behandlung unterworfen worden waren, während des gesamten Frühjahres und auch des Sommers frei von irgendwelchen Symptomen des Heuschnupfens oder Asthmas waren.

**Ansprüche**

1. Verwendung von Pollenextrakten, die nicht mehr als höchstens 5 Gew.-% an hochmolekularen Proteinen enthalten und die durch folgende Verfahren erhältlich sind:

Extraktion der Pollen mit einem wässrigen Extraktionsmittel,

Entfernung der hochmolekularen Proteine, vorzugsweise durch Abbau derselben, bis zur Erreichung des oben angegebenen Maximalgehaltes an diesen, sowie

Extraktion der Pollen mit einem nicht wässrigen Extraktionsmittel,

zur Herstellung von pharmazeutischen Präparaten zur prophylaktischen Behandlung von Allergien.

2. Verwendung nach Anspruch 1, dadurch gekennzeichnet, dass man den mit einem wässrigen Extraktionsmittel oder den mit einem nicht wässrigen Extraktionsmittel gewonnenen Extrakt verwendet oder dass man eine Mischung aus dem mit einem wässrigen Extraktionsmittel und dem mit einem nicht wässrigen Extraktionsmittel gewonnenen Extrakt zur Herstellung des pharmazeutischen Präparates verwendet.

3. Verwendung nach Anspruch 1 oder 2, dadurch gekennzeichnet, dass man Pollenextrakte verwendet, die durch das folgende Verfahren hergestellt sind:

(a) Extraktion der frisch geernteten oder getrockneten Pflanzenpollen mit einem wässrigen Extraktionsmittel,

(b) Abbau der vorhandenen hochmolekularen Proteine durch Einwirkung von Mikroorganismen zu niedrig molekularen Proteinen oder Aminosäuren bis zur Erreichung eines Maximalgehaltes an hochmolekularen Proteinen von 5 Gew.-%, bezogen auf das Trockengewicht,

(c) Abtrennung des wässrigen Mediums von den Pollenrückständen und

(d) Extraktion der Pollenrückstände mit einem nicht wässrigen Medium.

4. Verwendung nach Anspruch 3, dadurch gekennzeichnet, dass man aus dem nach den Verfahrensschritten (a), (b) und (c) gewonnenen wässrigen Extrakt das wässrige Extraktionsmittel abdampft oder dass man aus dem nach dem Verfahrensschritt (d) gewonnenen nicht wässrigen Extrakt das Extraktionsmittel abdampft und die entsprechenden konzentrierten Extrakte oder die Trockenrückstände dieser Extrakte zur Herstellung der pharmazeutischen Präparate verwendet.

5. Verwendung nach Anspruch 3 oder 4, dadurch gekennzeichnet, dass der im Verfahrensschritt (b) zum Abbau der hochmolekularen Proteine herangezogene Mikroorganismus ein Pilz der Gattung Mucor ist, vorzugsweise Mucor glomerata; Mucor luteus; Mucor mucedo; Mucor hiemalis oder Mucor alpinus.

6. Verwendung nach einem der Ansprüche 3 bis 5, dadurch gekennzeichnet, dass das im Verfahrensschritt (a) eingesetzte wässrige Extraktionsmittel eine Mischung aus Wasser und einem wasserlöslichen organischen Lösungsmittel ist, insbesondere einem niederen Alkohol oder niederen Keton, vorzugsweise Aethylalkohol oder Aceton, und
dass das im Verfahrensschritt (d) eingesetzte nicht wässrige Extraktionsmittel ein niederes Katon, ein Dialkyläther, ein Carbonsäureester oder ein aliphatischer oder cycloaliphatischer Kohlenwasserstoff ist, vorzugsweise Aceton, Diäthyläther, Petroläther oder Heptan.

7. Verwendung nach einem der Ansprüche 2 bis 6, dadurch gekennzeichnet, dass man eine Mischung aus einem gewichtsmässig grösseren Anteil, bezogen auf das Trockengewicht des Extraktes, des mit einem wässrigan Extraktionsmittel gewonnenen Extraktes und einem gewichtsmässig kleineren Anteil, bezogen auf das Trockengewicht des Extraktes, des mit einem nicht wässrigen Extraktionsmittel gewonnen Extraktes verwendet, vorzugsweise eine Mischung aus 10 bis 30 Gewichtsteilen, bezogen auf das Trockengewicht des Extraktes, des mit einem wässrigen Extraktionsmittel gewonnen Extraktes und einem Gewichtsteil, bezogen auf das Trockengewicht des Extraktes, des mit einem nicht wässrigen Extraktionsmittel gewonnen Extraktes.

8. Verwendung nach einem dar Ansprüche 1 bis 7, dadurch gekennzeichnet, dass als Pollenextrakt der Extrakt von Pollen verwendet wird, der von der Pflanzenfamilie der Nadelgehölze, insbesondere der Pinaceae, der Getreidearten und Gräser, nämlich der Gramineae, insbesondere der Klasse der Framineae, stammt, oder dass man einen Extrakt einer Mischung von Pollen dieser beiden Pflanzenfamilien oder einer Mischung von Pollen mindestens einer dieser Pflanzenfamilien mit Pollen aus anderen Pflanzenfamilien, insbesondere der Familien der Liliengewächse, der Korbblütler, der Rosengewächse, der Weidengewächse, Birkengewächse und Ulmengewächse verwendet.

9. Verwendung nach einem der Ansprüche 1 bis 8, dadurch gekennzeichnet, dass man die Pollenextrakte zur Herstellung von pharmazeutischen Präparaten zur oralen Verabreichung oder zur topischen Anwendung verwendet.

## Claims

1. A use of pollen extracts which contain at the most 5 % by weight of high-molecular weight proteins and which are obtainable by the following method:
   extraction of pollen with an aqueous extracting medium,
   removal of the high-molecular weight proteins, preferably by decomposition of the same, until the above indicated maximum content of these is achieved,
   as well as extraction of pollen with a non-aqueous extracting medium,
   for the manufacture of pharmaceutical preparations for prophylactic treatment of allergies.

2. A use according to claim 1, characterised in that the extract obtained with an aqueous extracting medium or the extract obtained with a non-aqueous extracting medium is used, or in that a mixture of the extract obtained from the aqueous extracting medium and the extract obtained from the non-aqueous extracting medium is used, for manufacturing the pharmaceutical preparation.

3. A use according to claim 1 or 2, characterised in that pollen extracts are used which are produced by the following method:
   a) extraction of freshly harvested or dried plant pollen with an aqueous extracting medium,
   b) decomposition of the high-molecular weight proteins present to low-molecular weight proteins or amino acids through the reaction of micro-organisms until achieving a maximum content of high-molecular weight proteins of 5 % by weight, relative to the dry weight,
   c) separation of the aqueous medium from the pollen residues and
   d) extraction of the pollen residues with a non-aqueous medium.

4. A use according to claim 3, characterised in that the aqueous extracting medium is evaporated from the aqueous extract obtained from stages (a), (b), and (c) of the method or in that the extracting medium is evaporated from the non-aqueous extract obtained from stage (d) of the method and the corresponding concentrated extracts or the dry residues of these extracts are used for manufacturing the pharmaceutical preparations.

5. A use according to claim 3 or 4, characterised in that the micro-organism employed in stage (b) of the method for decomposing the high-molecular weight proteins is a fungus of the mucor type, preferable Mucor glomerata; Mucor luteus; Mucor mucedo; Mucor hiemalis or Mucor alpinus.

6. A use according to one of claims 3 to 5, characterised in that the aqueous extracting medium used in stage (a) of the method is a mixture of water and a water-soluble organic solvent, in particular a low alcohol or low ketone, preferably ethyl alcohol or acetone, and

in that the non-aqueous extracting medium used in stage (d) of the method is a low ketone, a dialkyl ether, a carbonic acid ester or an aliphatic or cyclic aliphatic hydrocarbon, preferably acetone, diethyl ether, petroleum ether or heptane.

7. A use according to one of claims 2 to 6, characterised in that there is used a mixture of a larger proportion in terms of weight, relative to the dry weight of the extract, of the extract gained with an aqueous extracting medium and a smaller proportion in terms of weight, relative to the dry weight of the extract, of the extract gained with a non-aqueous extracting medium, preferably a mixture of 10 to 30 parts by weight, relative to the dry weight of the extract, of the extract gained with an aqueous extracting medium and one part by weight, relative to the dry weight of the extract, of the extract gained with a non-aqueous extracting medium.

8. A use according to one of claims 1 to 7, characterised in that as pollen extract the extract of pollen is used, which originates from the plant family of pine wood, in particular Pinaceae, of cereals and grasses, namely Gramineae, in particular the Framineae class, or that an extract is used of a mixture of pollen of both these plant families or a mixture of pollen of at least one of these plant families with pollen from other plant families, in particular liliaceous plants, composite flowers, rose plants, willow plants, birch plants and elm plants.

9. A use according to one of claims 1 to 8, characterised in that the pollen extracts are used for manufacturing pharmaceutical preparations for oral administering or for topical application.

**Revendications**

1. Utilisation d'extraits de pollen qui ne contiennent pas plus de 5% en poids de protéines de poids moléculaires élevés et que l'on peut obtenir par les procédés qui suivent :

extraction du pollen à l'aide d'un agent d'extraction aqueux, élimination des protéines de poids moléculaires élevés, de préférence par décomposition de celles-ci, jusqu'à l'obtention de la teneur maximale précitée en ces dernières,

comme aussi extraction du pollen à l'aide d'un agent d'extraction non aqueux,

en vue de la préparation de compositions pharmaceutiques destinées au traitement prophylactique d'allergies.

2. Utilisation suivant la revendication 1, caractérisée en ce que l'on utilise l'extrait obtenu à l'aide d'un agent d'extraction aqueux ou l'extrait obtenu à l'aide d'un agent d'extraction non aqueux, ou en ce que l'on utilise un mélange de l'extrait obtenu à l'aide d'un agent d'extraction aqueux et de l'extrait obtenu à l'aide d'un agent d'extraction non aqueux en vue de la préparation de la composition pharmaceutique.

3. Utilisation suivant la revendication 1 ou 2, caractérisée en ce que l'on utilise des extraits de pollen, que l'on a préparés par le procédé qui suit :

(a) extraction du pollen de plantes fraîchement récoltées ou séchées par un agent d'extraction aqueux,

(b) décomposition des protéines de poids moléculaires élevés présentes, sous l'effet de l'action de microorganismes, en protéines de faibles poids moléculaires ou en aminoacides jusqu'à l'obtention d'une teneur maximale en protéines de poids moléculaires élevés de 5% en poids, rapportés au poids à sec,

(c) séparation du milieu aqueux des résidus de pollen et

(d) extraction des résidus de pollen par un milieu non aqueux.

**4.** Utilisation suivant la revendication 3, caractérisée en ce que l'on chasse par évaporation l'agent d'extraction aqueux de l'extrait aqueux obtenu par mise en oeuvre des étapes opératoires (a), (b) et (c), ou en ce que l'on chasse par évaporation l'agent d'extraction de l'extrait non aqueux obtenu selon l'étape opératoire (d) et on utilise les extraits concentrés correspondants, ou les résidus secs de ces extraits pour la préparation des compositions pharmaceutiques.

**5.** Utilisation suivant la revendication 3 ou la revendication 4, caractérisée en ce que le microorganisme mis en oeuvre pour la décomposition des protéines de poids moléculaires élevés au cours de l'étape opératoire (b) est un champignon appartenant au genre Muco, de préférence Mucor glomerata, Muco luteus, Muco mucedo, Mucor hiemalis ou Mucor alpinus.

**6.** Utilisation suivant l'une quelconque des revendications 3 à 5, caractérisée en ce que l'agent d'extraction aqueux utilisé pour la mise en oeuvre de l'étape opératoire (a) est un mélange d'eau et d'un solvant organique soluble dans l'eau, plus particulièrement une cétone inférieure ou un alcool inférieur, de préférence l'acétone ou l'alcool éthylique

et en ce que l'agent d'extraction non aqueux utilisé pour la mise en oeuvre de l'étape opératoire (d) est une cétone inférieure, un éther dialkylique, un ester d'acide carboxylique, ou un hydrocarbure aliphatique ou cycloaliphatique, de préférence l'acétone, l'éther diéthylique, l'éther de pétrole ou l'heptane.

**7.** Utilisation suivant l'une des revendications 2 à 6, caractérisée en ce que l'on utilise un mélange d'une proportion pondéralement supérieure, par rapport au poids à sec de l'extrait, de l'extrait obtenu à l'aide d'un agent d'extraction aqueux et une proportion pondéralement plus faible, par rapport au poids à sec de l'extrait, de l'extrait obtenu à l'aide d'un agent d'extraction non aqueux, de préférence un mélange de 10 à 30 parties en poids, par rapport au poids à sec de l'extrait, de l'extrait obtenu à l'aide d'un agent d'extraction aqueux et une partie en poids, par rapport au poids à sec de l'extrait, de l'extrait obtenu à l'aide d'un agent d'extraction non aqueux.

**8.** utilisation suivant l'une des revendications 1 à 7, caractérisée en ce qu'à titre d'extrait de pollen, on utilise l'extrait de pollen qui provient de la famille des conifères, plus particulièrement des pinacées, des sortes ou espèces de céréales et d'herbes, notamment les graminées, plus particulièrement la classe des framinées, ou en ce que l'on utilise un mélange de pollen d'au moins une de ces familles de plantes et de pollen d'autres familles de plantes, plus particulièrement des familles des liliacées, des composées, des rosacées, des salicacées, des bétulacées et des ulmacées.

**9.** Utilisation suivant l'une des revendications 1 à 8, caractérisée en ce que l'on utilise les extraits de pollen pour la préparation de compositions pharmaceutiques destinées à l'administration par la voie orale ou à l'usage topique.